# EUROPEAN PATENT SPECIFICATION

(11) **EP 3 993 861 B1**
(45) Date of publication and mention of the grant of the patent: **31.01.2024**
(21) Application number: 20742490.4
(22) Date of filing: 02.07.2020
(51) Int. Cl.: A61M 16/08, A61M 16/20

(54) **DEVICE TO TRAP AND REMOVE A LIQUID PRESENT IN A CIRCUIT FOR VENTILATION OF A PATIENT**
VORRICHTUNG ZUM EINFANGEN UND ENTFERNEN EINER IN EINEM BEATMUNGSKREISLAUF EINES PATIENTEN VORHANDENEN FLÜSSIGKEIT
DISPOSITIF POUR PIÉGER ET ÉLIMINER UN LIQUIDE PRÉSENT DANS UN CIRCUIT DE VENTILATION D'UN PATIENT

(30) Priority: 04.07.2019 IT 201900010917
(43) Date of publication of application: 11.05.2022
(73) Proprietor: GVS S.p.A., 40069 Zola Predosa (Bologna) (IT)
(72) Inventor: BASSANI, Nicola, 40069 Zola Predosa (BO) (IT)
(74) Representative: Ripamonti, Enrico
(86) International application number: PCT/IB2020/056247
(87) International publication number: WO 2021/001777

(56) References cited:
- EP-A1- 1 342 485
- DE-A1- 3 643 624
- GB-A- 2 224 957
- US-A- 5 826 575
- US-A1- 2012 285 458

## Description

The subject of the present invention is a device to trap and remove condensate which forms in a circuit for ventilation of a patient.

As is known, in respiratory therapy, as well as during anaesthesia, devices are used which permit respiration of the patients. These devices supply to the patients various fluids such as gas, air or vapours, according to the needs. The fluids which are directed to the patient are advantageously filtered, heated and/or humidified such that, when they are inhaled by the patient, they have characteristics of temperature and humidity such as to make possible an ideal condition for the respiration or the pulmonary functionality of the patient, and they do not have a negative impact on the patient himself.

A problem which arises during said ventilation (either using systems which pressurise the fluid sent to the patient, or systems which send this fluid unpressurised to the patient) is the creation of condensation inside the tubing extending from the apparatus which carries out the ventilation to the patient and vice versa. This condensation can be created both because of the fluid sent to the patient, and because of the fluid exhaled by the patient and aspirated by the ventilation system.

The presence of this condensate can give rise to problems both for the ventilation apparatus and for the patient. As far as the ventilation apparatus is concerned, in the ventilation circuit there are present one or more anti-microbial filters disposed in the tubing connected to said apparatus, which filter(s) can prevent return into the apparatus of any drug emitted by the patient by respiration, or of any bacteria or other pathogenic elements derived from the patient. Any presence of humidity in the flow of fluid exhaled, or small drops of water including in spray form, can have a negative impact on the functionality of said filter. In fact, this humidity can give rise to incorrect readings of conventional electronic sensors of the ventilation apparatus, causing malfunctioning or blockages of the respiration system: in fact the alarm systems of this apparatus activate the blockage thereof as soon as they detect fluid coming from the respiration circuit.

On the other hand as far as the patient is concerned, any presence of condensation inside the tube which connects the patient to the ventilation device can have a negative impact on the rate of flow of the gas directed to the patient, and increase the resistance of the flow during the aspiration phase, and at the same time it can involve ingestion by the patient of condensate which may also contain harmful elements, such as bacteria which can be detrimental to the patient's health.

For this reason, it is known to provide a device to block and trap the liquid or condensate which forms inside the ventilation circuit comprising the ventilation apparatus and the tubing which is directed towards the patient, this filter being able to be used in both the inhalation and exhalation phases.

Various solutions are known which can prevent the creation of condensation, and thus the presence of condensate inside said circuit, which solutions are known as water traps, with manual or automatic emptying, disposed in various points of the respiration circuit.

DE3643624 describes a system for automatic, technically and hygienically safe elimination of the condensation which forms during the forced ventilation within a respiration circuit, with the lowest possible technical effort, financial expenditure and amount of care, without opening the ventilation system and modifying the ventilation parameters.

In the prior art, a water trap is described with a system for automatic removal of the condensation inserted in a respiration circuit, which trap is composed of various parts, i.e. a "T" connection with a supply nozzle, an output nozzle, and one nozzle turned back towards the bottom, to which an adapter with a filter-valve function is connected. The adapter is in the form of a hollow cylinder containing an absorbent material (paper) with a longitudinal lamellar capillary structure, which is retained mechanically on the bottom of the adapter. A bag for collection of the condensation is connected to this adapter.

In this way, during use of the respiration circuit, the condensed water gathers in the "T" connection and flows towards the adapter. The absorbent material which fills this adapter and receives the water has a capillary, lamellar structure with open pores. If it is dry, this material is permeable to gas. However, if it is wetted with the condensation water, the water is immediately aspirated by the absorbent material because of the above-described structure, and, after saturation of the liquid, the water is released into the collection bag. The resulting functionality of the known solution is thus that of a filter with a valve with automatic removal of the condensation, which simultaneously seals the ventilation system, thus avoiding losses of respiratory gas.

This prior art therefore describes absorption of the liquid which is present in the fluid circulating in the respiration circuit with simple condensation of this liquid within the "T" connection; the fluid moves freely in the circuit and within this connection, and nothing either limits this movement or facilitates the passage of the fluid containing the liquid towards the absorbent material which is present in the adapter. The capture of the condensation by the adapter takes place in a manner which is non-assisted and is "natural".

However, this involves a low level of absorption of the condensation: in fact, the amount which is absorbed is derived only from the contact of the liquid present in the fluid which moves in the respiration circuit with the "T" connection between the supply nozzle and the output nozzle, and only part of this liquid penetrates into the adapter provided with the absorbent material. Nothing in the known solution facilitates the contact of this fluid with the absorbent material present in the adapter by pushing this fluid towards said material.

US6105576 describes an apparatus, for tracheal use only, for treatment of respiratory gases, comprising a casing or container with a first opening which can be connected to a duct directed to the trachea of a patient, and a second opening which can be connected to a tube in which the respiratory fluid (for example air) passes, moved by a mechanical ventilation apparatus. Said container can receive and retain the liquid which passes through said openings, preventing it from reaching the ventilation apparatus or the patient.

Other known solutions are described in the patents US4090513, US4771770, US5042468 and US4516573. These prior arts describe devices which can heat/humidify the fluid directed towards the patient, and can simultaneously retain the condensate which is formed on the ventilation line to the patient. These devices comprise means placed in the ventilation circuit which are generally coated with hygroscopic substances which can obtain the aforementioned function, such as to prevent the formation and persistence of liquids inside the ventilation circuit.

However, said known solutions use hygroscopic materials which are soluble in water, and which can therefore be returned to the patient during the ventilation. The known solutions are also often complicated to produce, which involves a high cost for said devices.

EP265163 describes a device for heating and humidifying fluid directed to a patient, which device can be connected to a tracheal duct. This device contains a layer of polymer (polypropylene) which is electrostatically charged, and a second layer of pre-treated hydrophilic open-cell polyurethane foam, which can absorb the condensation present in the ventilation duct. US3881482 describes a similar solution. However, these solutions use layers of foam material which generally have excessive drops of pressure.

US5826575 describes a system for collecting the condensate associated with the fluid isolated from a patient subjected to ventilation.

This system comprises a casing containing a filter for the fluid exhaled by a patient, and being associated with an element for collecting the condensate present in this fluid. This element comprises a tube for discharging the condensate collected in the element itself. There is also a deflector present, to direct the condensate away from the filter and into the element for collection of the condensate.

In this known solution, the condensate is present in the element which collects it in liquid form, however it is not ensured that this liquid will be prevented from reaching the filter, the respiration apparatus or the patient, for example as a result of poor handling of the system.

The objective of the present invention is to provide a device to block and trap the condensate present inside a respiration duct of a ventilation circuit, which device is improved in comparison with the known solutions.

In particular, the objective of the invention is to provide a device of the aforementioned type which, in an optimum manner, can prevent the condensate present in a tube of a ventilation circuit from being able to reach the filter of the respiration apparatus, the apparatus itself, or the patient, thus detracting from the correct operation of the ventilation system.

Another objective is to provide a device of the aforementioned type which makes it possible with certainty to prevent the condensate from being able to reach the patient: in fact, by trapping the condensate, any movement thereof will be prevented. This takes place by directing the fluid exhaled (containing the liquid) in an appropriate manner to an area of the device where this condensed liquid can be removed from this fluid.

A further objective of the present invention is to provide a device of the aforementioned type which makes it possible to remove simply and completely the condensate which has gathered in the ventilation circuit.

Another objective is to prevent any type of bacterial contamination of the ventilation system and of the patient caused by the presence of condensate.

Another objective is to provide a device of the aforementioned type which is simple to produce and use by the medical operators who are using the ventilation apparatus in which this device is present.

These objectives and others, which will become apparent to persons skilled in the art, are achieved by a device to trap the liquid present inside a ventilation circuit according to claim 1.

For better understanding of the present invention, and purely by way of non-limiting indication, the following drawings are appended, in which:
figure 1 shows a perspective view of a first embodiment of a device according to the invention;
figure 2 shows a lateral view, with a part in cross-section, of the device in figure 1;
figure 3 shows a front view, partly in cross-section, of the device in figure 1;
figure 4 shows a view from above of the device in figure 1;
figure 5 shows a view from the rear of a variant of the device in figure 1; and
figure 6 shows a view in transverse cross-section along the line 6-6 in figure 5.

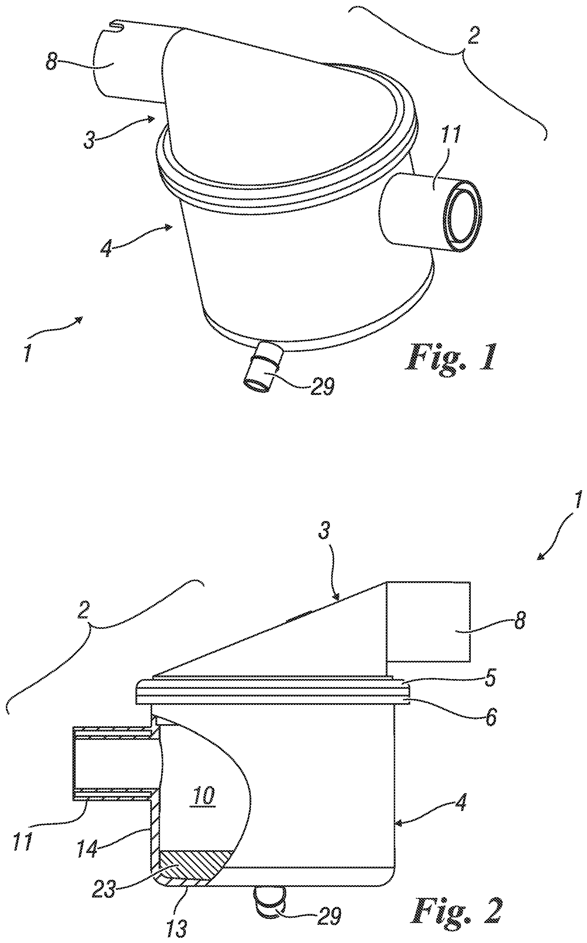

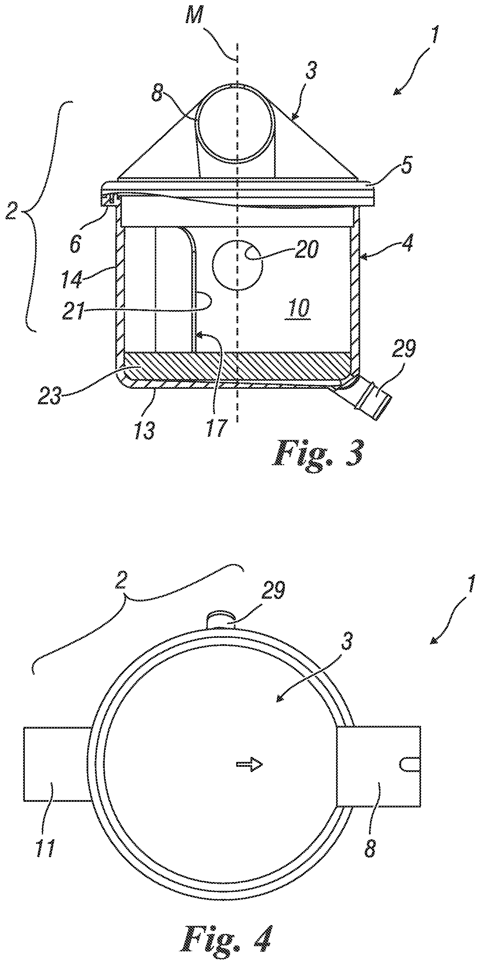

With reference to said figures, and in particular figures 1 to 4, a first embodiment is shown of a device 1 which can trap and remove a liquid inside a ventilation circuit of a patient, said circuit being of the type comprising a machine or apparatus for ventilation of the patient (not shown in the figures), and one or more tubes or ducts (also not shown) which can connect this machine or apparatus to the patient. This machine can be of the type used to ventilate a patient with assisted ventilation (patient capable of spontaneous respiration), or with controlled ventilation (patient who does not have spontaneous respiration, or has poor spontaneous respiration), or it can be an apparatus to ventilate a patient during a surgical intervention.

The device 1 is placed between the ventilation apparatus and the patient, and/or between the patient and the ventilation apparatus; this device comprises a body 2 defined by a first and a second portion 3 and 4 which are preferably removably coupled to one another: these portions can for example be connected by means of screwing or bayonet coupling at their free ends 5 and 6. It will be appreciated that the invention comprises a body 2 wherein the portions 3 and 4 are secured to one another, for example by means of welding.

The first portion 3, which in the figures is placed above the second portion 4, is hollow internally at 7, and preferably and advantageously contains an antimicrobial and hydrophobic filter (not shown) which can also protect the ventilation apparatus against the humidity present in the fluid which is exhaled by the patient and directed towards the apparatus itself. Said filter, which in itself is known, is usually placed between the portions 3 and 4, and will not be described further. This filter can also be placed between the device 1 and said apparatus.

The portion 3 comprises a projecting duct 8 to which there can be connected a ventilation tube (not shown) which is connected to the aforementioned ventilation apparatus. This portion 3 can also be connected directly to this apparatus via said duct 8.

The second portion 4 has the form of a bowl, it comprises an inner cavity 10, and has a projecting duct 11 to which there can be connected a second tube of the ventilation circuit (not shown) connected to a patient (and which tube collects the fluid exhaled by the patient).

The second portion also comprises a bottom wall 13 from which there rises a lateral wall 14, which, together with the wall 13, delimits the cavity 10. From the lateral wall 14 there extends at least one deflector or fin 17 extending inside the cavity 10, said deflector 17 being placed in the vicinity of an opening 20 in the aforementioned duct 11. Each deflector 17 can preferably extend from the wall 14 in such a way as not to be facing towards the longitudinal central axis M of the second portion 4, or such as to be inclined inside this portion towards said wall 14, at least with one of its end parts 21.

Each deflector has the active function of creating turbulence inside the portion 4, and of directing the flow of air in said portion in an appropriate way towards the bottom wall 13.

In addition or alternatively, each deflector or fin 17 can be formed such as to be inclined with respect to the bottom wall 13 (and not disposed on a plane orthogonal thereto); other deflectors or fins will be disposed vertically on the wall 14, and will also contribute towards directing the flow in an appropriate way towards the bottom wall 13.

According to a variant of the invention, in its part which faces towards the cavity 10, the wall 14 is formed with a plurality of projections, deformations or irregularities which project towards this cavity, and which themselves act as deflectors. The flow of air entering the opening 20 comes into contact with the wall 14 and its irregularities and is directed towards the bottom wall 13.

At this bottom wall 13 there is placed an absorbent element or absorbent pad 23 which can absorb any condensed liquid (water or other liquid comprising water vapour or medicinal vapour exhaled by the patient connected by means of a tube to the duct 11) which is present in the air entering from the opening 20 inside the cavity 10 during use of the ventilation circuit in which the device 1 is disposed.

Said pad 23 is a type of sponge, and is made mainly of a super-absorbent material such as a hydrophilic polymer material, for example open-cell polyurethane. This material is used in association with polyester fibre, and can absorb a quantity of water which is substantial, when compared with its own weight.

Preferably, the super-absorbent material is treated with antibacterial solutions which in themselves are known, such as chlorhexidine or the like. In addition, the super-absorbent material is advantageously treated with colouring solutions (medical grade), meaning that it changes colour as soon as the maximum absorption level has been reached.

In the solution in figures 1-4, the second portion 4 of the body 2 of the device 1 is also provided with a known duct 29 for discharge of the condensate to be released from the pad 23, or which may not have been completely absorbed by the pad.

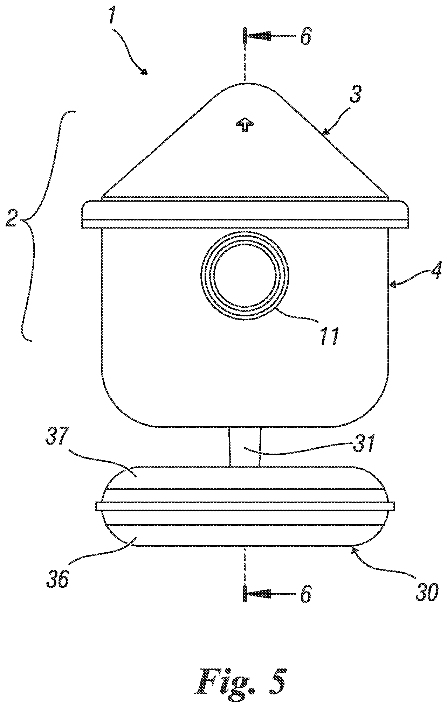

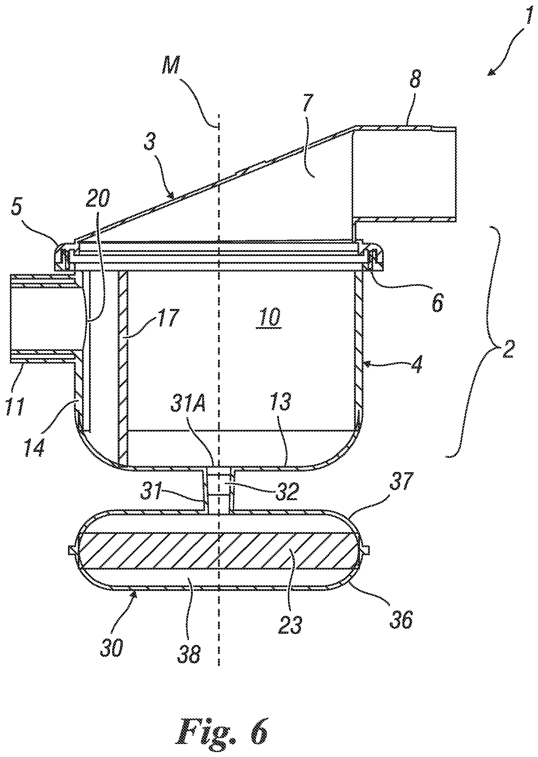

In the solution in figures 5 and 6, where parts corresponding to those already described in relation to figures 1-4 are indicated with the same numerical references, the device 1 comprises a part 30 for collection of the condensate which is "trapped" inside the chamber 10 to which this part 30 is connected externally by means of a tube 31. Preferably in this tube there is placed a non-return valve 32 (or a similar unit). This part 30 is preferably separable from the portion 4 (for example secured on the portion 4 by means of screwing together of threaded parts provided on this portion and on the part 30) .

The tube 31 can be placed in any position relative to the longitudinal axis M of the body 2 (and not only with an axis coinciding with that M, as in the figures), and can have any inclination relative to this axis. However, this tube still opens in the bottom wall 13 of the portion 4. This wall 13 can also be produced such as to be inclined towards the opening 31A in the tube 31 in this wall, or it can be recessed towards said opening 31A, such as to assist the passage of the condensate collected in the wall 13 (by means of the methods which will be described) towards the tube 31.

The collection part 30 is preferably formed such that it can be opened, and comprises at least two portions 36 and 37 which can be connected to one another for example by means of snap coupling, bayonet coupling, or by mutual sliding. The portion 37 is integral with the tube 31. These portions 36 and 37 can also be welded to one another, and in this case it will not be possible to open the collection part. In this last case in particular, it is thus possible to replace the absorbent element or pad 23 without stopping the ventilation circuit.

The two portions 36 and 37 define a closed cavity or chamber 38 inside the collection part 30 in which there is placed the pad 23 which can absorb and retain the condensate which is trapped in the device 1.

As in the case of the preceding figures 1-4, where the pad 23 can be extracted from the body 2 by separating the portions 3 and 4 (in the case in which they are not welded), the solution in figures 5-6 also makes it possible, by means of the separation of the parts 36 and 37, to remove the pad 23. Irrespective of whether the parts 36 and 37 are separable or not, the invention makes it possible to replace the collection part 30 when the pad 23 has reached the maximum limit of condensation absorbed. This part 30, which thus acts as a "cartridge", can be replaced by another part 30 with a new pad 23.

This operation of removal is however necessary after some hours (between 24 and 48) of use of the ventilation circuit even with the same patient (or it is carried out when the same ventilation apparatus is used at different times for two different patients).

It is now assumed that use is being made of the above-described ventilation circuit provided with the device 1 to trap and remove (from the circuit) condensate which forms in this circuit during use. In fact, it is known that, during the ventilation of a patient (during a surgical operation or to assist the patient's respiration), the fluid (air or gas) which is sent to the patient is also humidified. Or, the humidity itself of the environment in which the patient is placed is inhaled by the patient together with the air supplied by the ventilation apparatus.

When the patient exhales, part of the humidity which is present in the flow aspirated (or possibly taken by the patient himself from the environment in which he is located) returns to the ventilation apparatus. In order to prevent this humidity, which condenses in the piping of the ventilation circuit, from being able to reach the filter present in this circuit before entering said apparatus or the ventilation apparatus itself, or from being able to be returned to the patient, the device 1 is provided, placed before said apparatus (and the aforementioned filter, if it is not associated with this device) along the path which the air emitted by the patient follows when returning to this apparatus.

The air exhaled reaches the body 2 through the duct 11 and penetrates into the cavity 10 before exiting therefrom through the duct 8. Inside this cavity 10, the flow of the fluid exhaled by the patient interferes with the deflector means defined by each fin 17 (in whichever way it is produced, i.e. as a single fin or a plurality of pins projecting from the wall 14 of the portion L, or as irregularities and deformations of the wall 14 itself). This interference also means that said fluid, without however increasing the pressure inside the chamber 10, has its own speed slowed down before it passes to the duct 8, by following a "forced" path inside said chamber 10, before reaching this duct; this is obtained thanks to the contact between said flow of fluid and each fin or deflector 17 present in said cavity (which creates turbulence within the body 2), and thanks to a minimum internal volumetric dimension (of the cavity 10) of the body 2, which ensures this slowing down, and elongation of the path in the chamber 10. Said internal dimension or volume is at least 2000 mm³ from the opening 20 as far as the wall which faces it.

Thanks to at least one from out of the fins and the distance between the opening 20 and the wall part of the body 2 which faces it (if this body 2 had a circular cross-section, this distance would represent the diameter of the cross-section), the flow of fluid is deflected and necessarily tends to move from the opening 20 towards the wall 13 of the body 2. This can also be assisted by appropriate inclination of the fin(s) 17 with respect to this wall 13.

In this way, the flow of fluid (wetness) laps the pad 23, such that the humidity (i.e. the liquid) in this fluid content can be absorbed by this pad. In the case of the solution in figures 5 and 6, the fluid lapping the wall 13 (which is colder than the fluid itself) has its speed slowed down, making possible the depositing along this wall 13 of the humidity, which condenses and then flows into the tube 31, in order to reach the cavity 38 of the collection part 30. Here the fluid reaches the pad 23 and is absorbed by it.

It should be noted that also the contact itself with the lateral wall 14 leads to direct condensation of the humidity inside the body 2, and then dropping of the condensate towards the bottom wall 13 of this body, with the consequences indicated above.

When the pad is full of condensate (or after a predetermined time of use of the device 1), it is extracted from the body 2 and replaced. Alternatively, the entire body 2 is replaced in the case in which its portions 3 and 4 are welded to one another.

As stated, in order to show how much condensate has been absorbed by the pad 23, the pad can contain a material which changes colour when the quantity of condensate collected increases. For example, this material is copper sulphate, which gradually turns blue as it absorbs humidity.

It should also be noticed that the known antimicrobial hydrophobic filter also contributes to stopping the humidity which condenses on said filter, and then falls in the form of drops towards the wall 13 of the body 2, thus being absorbed by the pad 23.

Thanks to the invention, a device is obtained which extracts the humidity from the flow of air exhaled by the patient, such as to avoid problems in the correct functioning of the hydrophobic filter placed before the ventilation apparatus on the path of the exhaled flow (for example also inside the body 2, as described), in the functioning of the apparatus itself, and prevents the condensate from being able to return to the patient.

According to the invention, a device is thus obtained which actively controls the flow of fluid coming from the patient, and "guides" it on a defined path. In fact, this fluid is subjected to forced guiding by means of the fins 17 (or equivalent deflector means) on a path which generates turbulence ensuring that the flow comes into direct contact with the absorbent pad, thus making the absorption capacity of the pad very high.

The form of the wall 14 which defines the forced path for said fluid, after having brought the flow into direct contact with the absorbent pad, channels the fluid towards the output duct 8. During this movement, the fluid meets the antimicrobial hydrophobic filter, which blocks the residue of humidity remaining in the flow, and at the same time filters the flow being output.

The result of this passage of fluid in the body 2 is not only a flow free from humidity, but also a flow which is free from humidity and is filtered. This will mean that problems of cross-contamination are avoided, as well as alarms caused by the humidity in the machine. Thus, the device according to the invention operates efficiently thanks to the combination of various elements which all contribute to the aim of filtering of the fluid.

In fact:
a - the deflector means 17 and the dimensions of the cavity 10 determine the path of the flow by means of the turbulence which is generated in this cavity;
b - the absorbent pad can absorb a large quantity of humidity, thanks to the new path of the flow determined by the deflector means;
c - the efficiency of the hydrophobic filter at the output is increased thanks to the path of the flow imposed by the deflector means.

It should be noted that the device 1 operates efficiently in any position, i.e. parallel to the ground rather than inclined or overturned. There are no positions in which the filter cannot perform at its best.

Various embodiments of the invention have been described. However, others are also possible in the light of the preceding description, provided that they remain within the scope of the invention defined by the following claims.

## Claims

1. Device which can trap and remove a condensate derived from a fluid which is exhaled by a patient connected to a ventilation circuit, said device (1) comprising a body (2) with a first duct (8) for direct or indirect connection to a ventilation apparatus of this circuit, and a second duct (11) configured to be connected to the patient, said body (2) having a first and a second portion (3, 4) connected to one another, the second portion (4) being able to collect said condensate from the exhaled fluid, said second portion comprising a bottom wall (13) from which there rises a lateral wall (14) delimiting a cavity (10) for collection of said condensate, said lateral wall (14) having an opening (20) connected to said second duct (11) for intake of the fluid exhaled into said cavity (10), an absorbent element (23) being provided which can absorb this condensate collected in the second portion (4) of this body (2), said absorbent element (23) being replaceable, wherein said body (2) comprises deflector means (17) placed inside said cavity (10) in the second portion (4) and which means can direct the flow of the exhaled fluid entering into this cavity (10) towards the absorbent element (23).

2. Device according to claim 1, **characterised in that** said deflector means are at least one fin (17) projecting from the lateral wall (14) of the second portion (4) towards the inside of the cavity (10) of this second portion.

3. Device according to claim 1, **characterised in that** said deflector means are irregularities or deformations of the lateral wall (14) inside the cavity (10) of said body (2) of the device, with said irregularities or projections coordinating the flow of the fluid, the fluid being directed by said irregularities or projections towards the absorbent element (23).

4. Device according to claim 1, **characterised in that** said deflector means (17) are formed such as to direct the flow of aspirated fluid entering into the body (2) of the device towards the bottom wall (13) of said second portion (4) of body (2).

5. Device according to claim 4, **characterised in that** said absorbent element (23) is placed on the bottom wall (13) of said second portion (4).

6. Device according to claim 5, **characterised in that** it comprises a discharge duct (29) which is connected to said bottom wall (13) of the second portion (4) of said body (2) of the device.

7. Device according to claim 4, **characterised in that** it comprises a part (30) for collection of said liquid, which part is connected externally to the bottom wall (13) of said second portion (4) by means of a tube (31), said collection part (30) comprising a closure chamber or cavity (38) which can receive said liquid from the second portion (4) of said body (2), said chamber or cavity (38) containing the absorbent element (23).

8. Device according to claim 7, **characterised in that** the bottom wall (13) converges towards said tube (31).

9. Device according to claim 7, **characterised in that** said collection part (30) is separable from the second portion (4) of said body (2), said collection part preferably comprising two portions (36, 37) which are coupled and define said chamber or cavity (38).

10. Device according to claim 9, **characterised in that** said portions (36, 37) are alternatively secured to one another or detachably connected to one another.

11. Device according to claim 1, **characterised in that** the absorbent element (23) is alternatively replaceable by means of autonomous removal from said body (2), by means of entire replacement of said body, or by means of replacement of a part of said body (2).

12. Device according to claim 1, **characterised in that** the body (2) of the device (1) has an internal volume equal to at least 2000 mm³.

13. Device according to claim 1, **characterised in that** said absorbent element (23) comprises a super absorbent.

14. Device according to claim 1, **characterised in that** said absorbent element (23) is treated with an antibacterial solution.

15. Device according to claim 1, **characterised in that** said absorbent element (23) contains a material which shows the quantity of condensate absorbed.

16. Device according to claim 1, **characterised in that** an antimicrobial and hydrophobic filter is present inside the body (2) of the device, between said first and second portions (3, 4).

## Patentansprüche

1. Vorrichtung zum Auffangen und Entfernen von Kondensat aus einem Fluid, das von einem Patienten ausgeatmet wird, der mit einem Beatmungskreislauf verbunden ist, die Vorrichtung (1) umfassend einen Körper (2) mit einer ersten Leitung (8) zur direkten oder indirekten Verbindung mit einem Beatmungsgerät dieses Kreislaufs und eine zweite Leitung (11), die konfiguriert ist, um mit dem Patienten verbunden zu werden, wobei der Körper (2) einen ersten und einen zweiten Abschnitt (3, 4) aufweist, die miteinander verbunden sind, wobei der zweite Abschnitt (4) in der Lage ist, das Kondensat dem ausgeatmeten Fluid zu sammeln, der zweite Abschnitt umfassend eine Bodenwand (13), von der eine Seitenwand (14) aufsteigt, die einen Hohlraum (10) zum Sammeln des Kondensats begrenzt, wobei die Seitenwand (14) eine Öffnung (20) aufweist, die mit der zweiten Leitung (11) verbunden ist, um das ausgeatmete Fluid in den Hohlraum (10) zu leiten, wobei ein absorbierendes Element (23) bereitgestellt ist, das dieses Kondensat aufnehmen kann, das in dem zweiten Abschnitt (4) dieses Körpers (2) gesammelt wird, wobei das absorbierende Element (23) austauschbar ist, wobei der Körper (2) Ablenkeinrichtungen (17) umfasst, die im Inneren des Hohlraums (10) in dem zweiten Abschnitt (4) platziert sind und wobei die Einrichtungen den Strom des ausgeatmeten Fluids, das in diesen Hohlraum (10) eintritt, in Richtung des absorbierenden Elements (23) lenken können.

2. Vorrichtung nach Anspruch 1, **dadurch gekennzeichnet, dass** die Ablenkeinrichtungen mindestens eine Rippe (17) sind, die von der Seitenwand (14) des zweiten Abschnitts (4) in das Innere des Hohlraums (10) dieses zweiten Abschnitts hervorsteht.

3. Vorrichtung nach Anspruch 1, **dadurch gekennzeichnet, dass** die Ablenkeinrichtungen Unregelmäßigkeiten oder Verformungen der Seitenwand (14) innerhalb des Hohlraums (10) des Körpers (2) der Vorrichtung sind, wobei die Unregelmäßigkeiten oder Vorsprünge die Strömung des Fluids koordinieren, wobei das Fluid durch die Unregelmäßigkeiten oder Vorsprünge in Richtung des absorbierenden Elements (23) gelenkt wird.

4. Vorrichtung nach Anspruch 1, **dadurch gekennzeichnet, dass** die Ablenkeinrichtungen (17) gebildet sind, um den Strom des angesaugten Fluids, das in den Körper (2) der Vorrichtung eintritt, zu der Bodenwand (13) des zweiten Abschnitts (4) des Körpers (2) zu lenken.

5. Vorrichtung nach Anspruch 4, **dadurch gekennzeichnet, dass** das absorbierende Element (23) an der Bodenwand (13) des zweiten Abschnitts (4) platziert ist.

6. Vorrichtung nach Anspruch 5, **dadurch gekennzeichnet, dass** sie eine Auslassleitung (29) umfasst, die mit der Bodenwand (13) des zweiten Abschnitts (4) des Körpers (2) der Vorrichtung verbunden ist.

7. Vorrichtung nach Anspruch 4, **dadurch gekennzeichnet, dass** sie ein Teil (30) zum Sammeln des Fluids umfasst, das außen mittels eines Rohrs (31) mit der Bodenwand (13) des zweiten Abschnitts (4) verbunden ist, das Sammelteil (30) umfassend eine Verschlusskammer oder einen Hohlraum (38), die/der das Fluid aus dem zweiten Abschnitt (4) des Körpers (2) aufnehmen kann, wobei die Kammer oder der Hohlraum (38) das absorbierende Element (23) enthält.

8. Vorrichtung nach Anspruch 7, **dadurch gekennzeichnet, dass** die Bodenwand (13) in Richtung des Rohrs (31) konvergiert.

9. Vorrichtung nach Anspruch 7, **dadurch gekennzeichnet, dass** das Sammelteil (30) von dem zweiten Abschnitt (4) des Körpers (2) trennbar ist, das Sammelteil vorzugsweise umfassend zwei Abschnitte (36, 37), die gekoppelt sind und die Kammer oder den Hohlraum (38) definieren.

10. Vorrichtung nach Anspruch 9, **dadurch gekennzeichnet, dass** die Abschnitte (36, 37) abwechselnd aneinander befestigt oder lösbar miteinander verbunden sind.

11. Vorrichtung nach Anspruch 1, **dadurch gekennzeichnet, dass** das absorbierende Element (23) alternativ durch selbständiges Entfernen aus dem Körper (2), durch vollständiges Ersetzen des Körpers oder durch Ersetzen eines Teils des Körpers (2) austauschbar ist.

12. Vorrichtung nach Anspruch 1, **dadurch gekennzeichnet, dass** der Körper (2) der Vorrichtung (1) ein Innenvolumen gleich wie mindestens 2000 mm³ aufweist.

13. Vorrichtung nach Anspruch 1, **dadurch gekennzeichnet, dass** das absorbierende Element (23) ein Superabsorbens umfasst.

14. Vorrichtung nach Anspruch 1, **dadurch gekennzeichnet, dass** das absorbierende Element (23) mit einer antibakteriellen Lösung behandelt ist.

15. Vorrichtung nach Anspruch 1, **dadurch gekennzeichnet, dass** das absorbierende Element (23) ein Material enthält, das die Menge des absorbierten Kondensats zeigt.

16. Vorrichtung nach Anspruch 1, **dadurch gekennzeichnet, dass** ein antimikrobieller und hydrophober Filter im Inneren des Körpers (2) der Vorrichtung zwischen dem ersten und dem zweiten Abschnitt (3, 4) vorhanden ist.

## Revendications

1. Dispositif destiné au piégeage et à l'élimination d'un condensat provenant d'un fluide exhalé par un patient branché à un circuit de ventilation, ledit dispositif (1) comprenant un corps (2) avec un premier conduit (8) pour être connecté directement ou indirectement à un appareil de ventilation de ce circuit, et un deuxième conduit (11) configuré pour être branché sur le patient, ledit corps (2) ayant une première et une deuxième partie (3, 4) reliées l'une à l'autre, la deuxième partie (4) pouvant recueillir ledit condensat du fluide exhalé, ladite deuxième partie comprenant une paroi de fond (13) de laquelle s'élève une paroi latérale (14) délimitant une cavité (10) destinée à la collecte dudit condensat, ladite paroi latérale (14) présentant une ouverture (20) reliée audit deuxième conduit (11) d'admission du fluide exhalé dans ladite cavité (10), un élément absorbant (23) pouvant absorber ce condensat recueilli dans la deuxième partie (4) de ce corps (2) étant prévu, ledit élément absorbant (23) étant remplaçable, dans lequel ledit corps (2) comprend des moyens déflecteurs (17) placés à l'intérieur de ladite cavité (10) dans la deuxième partie (4) et qui peuvent diriger le flux du fluide expiré entrant dans cette cavité (10) vers l'élément absorbant (23).

2. Dispositif selon la revendication 1, **caractérisé en ce que** lesdits moyens déflecteurs sont au moins une ailette (17) faisant saillie de la paroi latérale (14) de la deuxième partie (4) vers l'intérieur de la cavité (10) de cette deuxième partie.

3. Dispositif selon la revendication 1, **caractérisé en ce que** lesdits moyens déflecteurs sont des irrégularités ou des déformations de la paroi latérale (14) à l'intérieur de la cavité (10) dudit corps (2) du dispositif, lesdites irrégularités ou projections coordonnant l'écoulement du fluide, le fluide étant dirigé par lesdites irrégularités ou projections vers l'élément absorbant (23).

4. Dispositif selon la revendication 1, **caractérisé en ce que** lesdits moyens déflecteurs (17) sont formés de manière à diriger le flux de fluide aspiré entrant dans le corps (2) du dispositif vers la paroi de fond (13) de ladite deuxième partie (4) du corps (2).

5. Dispositif selon la revendication 4, **caractérisé en ce que** ledit élément absorbant (23) est placé sur la paroi inférieure (13) de ladite deuxième partie (4).

6. Dispositif selon la revendication 5, **caractérisé en ce qu'**il comprend un conduit d'évacuation (29) qui est relié à ladite paroi inférieure (13) de la deuxième partie (4) dudit corps (2) du dispositif.

7. Dispositif selon la revendication 4, **caractérisé en ce qu'**il comprend une partie (30) de collecte dudit liquide, laquelle partie est reliée extérieurement à la paroi de fond (13) de ladite deuxième partie (4) au moyen d'un tube (31), ladite partie de collecte (30) comprenant une chambre de fermeture ou une cavité (38) pouvant recevoir ledit liquide de la deuxième partie (4) dudit corps (2), ladite chambre ou cavité (38) contenant l'élément absorbant (23).

8. Dispositif selon la revendication 7, **caractérisé en ce que** la paroi inférieure (13) converge vers ledit tube (31).

9. Dispositif selon la revendication 7, **caractérisé en ce que** ladite partie de collecte (30) est séparable de la deuxième partie (4) dudit corps (2), ladite partie de collecte comprenant de préférence deux parties (36, 37) qui sont couplées et définissent ladite chambre ou cavité (38).

10. Dispositif selon la revendication 9, **caractérisé en ce que** lesdites parties (36, 37) sont alternativement fixées l'une à l'autre ou reliées l'une à l'autre de manière amovible.

11. Dispositif selon la revendication 1, **caractérisé en ce que** l'élément absorbant (23) est alternativement remplaçable par retrait autonome dudit corps (2), par remplacement complet dudit corps, ou par remplacement d'une partie dudit corps (2).

12. Dispositif selon la revendication 1, **caractérisé en ce que** le corps (2) du dispositif (1) a un volume interne égal à au moins 2000 mm³.

13. Dispositif selon la revendication 1, **caractérisé en ce que** ledit élément absorbant (23) comprend un super absorbant.

14. Dispositif selon la revendication 1, **caractérisé en ce que** ledit élément absorbant (23) est traité avec une solution antibactérienne.

15. Dispositif selon la revendication 1, **caractérisé en ce que** ledit élément absorbant (23) comprend un matériau qui indique la quantité de condensat absorbée.

16. Dispositif selon la revendication 1, **caractérisé en ce qu'**un filtre antimicrobien et hydrophobe est présent à l'intérieur du corps (2) du dispositif, entre lesdites première et deuxième parties (3, 4).
